(19) Europäisches Patentamt
European Patent Office
Office européen des brevets

(11) Veröffentlichungsnummer: **0 063 266**
**B1**

(12) # EUROPÄISCHE PATENTSCHRIFT

(45) Veröffentlichungstag der Patentschrift: **03.07.85**

(51) Int. Cl.[4]: **A 61 K 9/22,** A 61 K 9/24, A 61 K 9/54

(21) Anmeldenummer: **82102695.2**

(22) Anmeldetag: **31.03.82**

(54) **Galenische Zubereitung.**

(30) Priorität: **07.04.81 DE 3113901**

(43) Veröffentlichungstag der Anmeldung: **27.10.82 Patentblatt 82/43**

(45) Bekanntmachung des Hinweises auf die Patenterteilung: **03.07.85 Patentblatt 85/27**

(84) Benannte Vertragsstaaten: **AT BE CH DE FR GB IT LI LU NL SE**

(56) Entgegenhaltungen:
**DE - A - 1 467 781**
**DE - A - 2 030 501**
**US - A - 3 261 859**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Einig, Heinz, Dr., Aspenweg 12, D-6730 Neustadt (DE)**
Erfinder: **Buehler, Volker, Dr., Liebigstrasse 2, D-7500 Karlsruhe (DE)**
Erfinder: **Koch, Winfried, Dr., Bibienastrasse 5, D-6830 Schwetzingen (DE)**
Erfinder: **Schwarz, Joachim Arthur, Dr., Am Dorfzaun 2, D-6701 Ruchheim (DE)**

**EP 0 063 266 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft eine neue galenische Zubereitung, welche einen calciumantagonistischen Wirkstoff und gegebenenfalls weitere Wirkstoffe enthält.

In der medikamentösen Therapie ist der Einsatz von oralen galenischen Arzneiformen mit verzögerter Wirkstoff-Freisetzung der wirksamen Bestandteile allgemein gebräuchlich und insbesondere erforderlich, um die Wirkungsdauer von Arzneistoffen, besonders von Arzneistoffen mit kürzeren biologischen Halbwertszeiten, zu verlängern. Orale Arzneiformen mit verzögerter Freisetzung der Wirkstoffe erhöhen die Patienten-Compliance beträchtlich, da durch geringere Einnahmefrequenz gegenüber üblichen Arzneiformen mit nichtverzögerter Wirkstoff-Freisetzung eine grössere Sicherheit bei der Therapie gewährleistet wird. Ausserdem lassen sich durch orale Arzneiformen mit verzögerter Freisetzung der Wirkstoffe vielfach unerwünschte Spitzen der Arzneimittelkonzentration im Plasma vermeiden.

Retard-Arzneiformen sollen eine Initialphase mit schneller Wirkstoff-Freisetzung und eine Erhaltungsphase besitzen, um eine Verlängerung des therapeutisch erwünschten Plasmaspiegels zu erhalten. Die Initialphase ist unbedingt erforderlich, damit möglichst rasch der erforderliche Plasmaspiegel erreicht wird. Diese Form der Retard-Zubereitung wird als ideal bezeichnet, siehe dazu Thieme Verlag, Stuttgart, Pharmazeutische Technologie 1978, Seite 487. Vielfach werden auch Retard-Arzneiformen — vornehmlich solche, die als Langzeit- oder Dauertherapeutika eingesetzt werden — angewendet, die keine Initialdosis besitzen, sondern nur die Erhaltungsdosis. Die Erhaltungsdosis wird gleichmässig und verzögert freigesetzt.

In Abhängigkeit von der biologischen Halbwertszeit stellt sich bei wiederholter Einnahme eines Medikamentes ein Fliessgleichgewicht (Steady state) zwischen aus dem Plasma eliminierten und in das Plasma aufgenommenen Wirkstoff ein.

Zum Erreichen von steady-state-Konzentrationen in therapeutisch notwendiger Höhe ist bei Substanzen mit kurzen Halbwertszeiten eine häufige Applikation erforderlich, was aus Gründen der Patienten-Compliance unerwünscht ist.

Eine ähnliche Situation liegt bei Substanzen mit längerer Halbwertszeit vor, bei denen ein überwiegender Anteil vor Erreichen des grossen Körperkreislaufs verstoffwechselt wird (first-pass-Effekt im Darm und/oder in der Leber). Allgemein werden daher Retard-Arzneiformen mit Substanzen mit ausgeprägtem first-pass-Effekt als sehr kritisch betrachtet [Der Internist *19*, 333 (1978), Pharm Ind. *40*, 374 (1978), besonders Seite 381, linke Spalte].

Substanzen mit ausgeprägtem first-pass-Effekt sind eine Reihe von β-Rezeptorenblockern, wie beispielsweise Propranolol, oder Calciumantagonisten, wie z.B. Verapamil und Gallopamil.

Die US-PS 4 248 858 beschreibt eine neue Retard-Zubereitung von Propranolol. Danach liegen 160 mg Propranolol in einem Pressling vor, dessen Wirkstoffliberation durch Einbettung in Gelbildner und unlösliche Polymere retardiert. Der Pressling ist von einer äusseren Drageeschicht, die weitere 20 mg Propranolol mit schneller Wirkstoff-Freisetzgeschwindigkeit enthält, durch einen magensaftresistenten Film getrennt, der vermeiden soll, dass der Wirkstoff des retardierten Presslings bereits im Magen freigesetzt wird.

Diese Form eignet sich nur für Substanzen wie Propranolol, das bei der ersten Leberpassage 4-Hydroxypropranolol bildet, dessen Wirksamkeit ähnlich der von Propranolol selbst ist [Brit. J. Pharmacy (1971) *43*, 222].

Gegenstand der Erfindung ist eine galenische Zubereitung, welche einen calciumantagonistischen Wirkstoff und gegebenenfalls weitere Wirkstoffe enthält, zur oralen Applikation mit prolongierter Wirkung zur Dauertherapie enthaltend nur die Erhaltungsdosis des Wirkstoffes an galenische Hilfsstoffe gebunden, dadurch gekennzeichnet, dass sie als calciumantagonistischen Wirkstoff Gallopamil oder Verapamil enthält und dass bei der Erhaltungsdosis das Verhältnis der Anteile mit schneller Wirkstoff-Freisetzung zu den Anteilen mit langsamer Wirkstoff-Freisetzung im Bereich von 1 : 0,6 bis 1 : 6 liegt.

Verapamil (X = H) und Gallopamil (X = $OCH_3$) besitzen die Formel

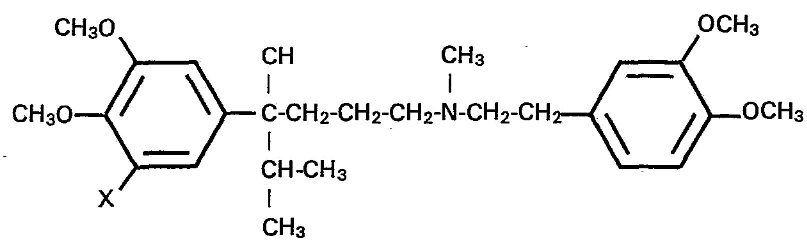

Die Substanzen werden in der Arzneiform vorzugsweise als Hydrochloride eingesetzt.

Neben diesen Substanzen können die neuen Zubereitungen weitere Wirkstoffe wie Diuretika, Psychopharmaka und Hypotensiva enthalten. Diese zusätzlichen Wirkstoffe liegen je nach der Grösse ihrer biologischen Halbwertszeit in der Phase mit schneller und/oder langsamer Wirkstoff-Freisetzung vor.

Das Verhältnis von sich schnell freisetzendem Verapamil bzw. Gallopamil zu langsam freiwerdendem Wirkstoff in der galenischen Zubereitung liegt zwischen 1 : 0,6 und 1 : 6, vorzugsweise zwischen 1 : 1 und 1 : 4, insbesondere zwischen 1 : 1,2 und 1 : 3.

Durch dieses Verhältnis wird bei Dosierung in der richtigen Menge sichergestellt, dass weitgehend

konstante und therapeutisch sinnvolle Plasmaspiegel erhalten werden, wobei die Wirkstoffe in der Magen-Dünndarm-Passage-Zeit (in der Regel 3 bis 5 Stunden) freigesetzt werden.

Die Herstellung der galenischen Zubereitung kann in an sich bekannter Weise erfolgen. So lassen sich Mantel- und Schichttabletten, bei denen eine Schicht bzw. der Kern der Manteltablette aus retardiertem Granulat und eine weitere Schicht bzw. der umhüllende Teil der Manteltablette aus schnell den Wirkstoff freisetzenden Granulat darstellt, oder Retard-Dragees, bei denen der retardierte Kern mit einer wirkstoffhaltigen Zuckerschicht mit schneller Freisetzung dragiert ist, nach allgemein üblichen und gängigen Verfahren herstellen.

Ein Granulat mit schneller Wirkstoff-Freisetzung erhält man durch Granulation des Wirkstoffes mit Zucker, Zellulose, Stärke und Bindemittel und Zusatz von Trennmitteln und Fliessregulierstoffen zum Granulat. Die Wirkstoffanteile können im Granulat weitgehend variiert werden. Vorzugsweise liegen sie bei 40 bis 80%. Bei Verwendung dieser Granulate als umhüllendes Material für Manteltabletten oder beim Einsatz in Zweischichttabletten zeigte sich, dass die Geschwindigkeit der Wirkstoff-Freisetzung weitgehend unabhängig von dem Druck bei der Pressung der Tablette ist.

Granulate mit verzögerter Wirkstoff-Freisetzung können mit Hilfe von Gelbildnern erhalten werden, die sich im Verdauungstrakt nicht lösen, sondern Quellkörper bilden, aus dem die Wirkstoffe langsam in die Verdauungsflüssigkeiten diffundieren.

Als Gelbildner eignen sich besonders Alginate — wie Natriumalginat — insbesondere solche, deren 10%ige wässrige Lösungen bei 20°C eine Viskosität von 100 bis 3000 cp besitzen. Beim Einsatz von niederviskosen Alginaten empfiehlt sich der Zusatz von wasserunlöslichen Naturstoffen — wie Cellulosen oder Stärken —, beim Einsatz von hochviskosen Alginaten der Zusatz von löslichen Stoffen — wie Lactose oder niedermolekularen Polyethylenglykolen. Auch durch Einsatz von Polyacryl- und Polymethacryl-Säurederivaten lassen sich sehr gute Freisetzungsgeschwindigkeiten erzielen. Auch der Pressdruck beim Tablettieren kann bei den Granulaten mit verzögerter Wirkstoff-Freisetzung die Freisetzung des Wirkstoffes beeinflussen.

Es ist auch möglich, die Wirkstoffe in Granulaten oder Pellets mit verzögerter Wirkstoff-Freisetzung zusammen mit den freien bzw. nicht retardierten Wirkstoffen in eine therapeutische Einheit — z.B. Steckkapseln — zu füllen.

Durch die Erfindung wird es möglich, Arzneiformen zu schaffen, die Verapamil oder Gallopamil enthalten und die über einen langen Zeitraum einen konstanten und effektiven Wirkspiegel erzielen.

Die neue Retard-Arzneiform eignet sich hervorragend zum Einsatz als Dauertherapeutikum, wobei über den gesamten Therapiezeitraum relativ enge und therapeutisch wirksame Plasmaspiegel erzielt werden. Ein Absinken des Plasmaspiegels in subtherapeutische Bereiche ist bei regelmässiger Einnahme nicht zu befüchten. Darüber hinaus wird durch die neue Retard-Form die Applikationsfrequenz auf 1 bis 2 × täglich reduziert, wodurch die Patienten-Compliance deutlich erhöht wird. Auch die Arzneimittelsicherheit wird erhöht.

Schliesslich lässt sich die neue Zubereitung sehr einfach und mit Hilfe üblicher Verfahren herstellen.

*Beispiel 1*

A. Herstellung der Ausgangsgranulate

a) Granulat mit schneller Wirkstoff-Freisetzung

10,6 kg Verapamil, 4 kg Cellulosepulver, 0,6 kg ®Kollidon VA 64 und 4 kg Lactose wurden innigst miteinander vermischt, dann unter Zugabe von 6 l Wasser unter Rühren befeuchtet und maschinell durch starkes Rühren und Kneten zu einer erdfeuchten Masse verarbeitet, die durch Hindurchpressen durch ein Sieb zu einem Granulat verarbeitet wurde. Nach Trocknen des Granulats wurde erneut gesiebt. Durch Zumischen von 0,7 kg Cellulosepulver und 0,1 kg Magnesiumstearat erhielt man ein pressfähiges Granulat.

b) Granulat mit langsamer Wirkstoff-Freisetzung

14 kg Verapamil, 25 kg Natriumalginat, 4 kg ®Kollidon 30 (kettenförmiges PVP, M = 30 000) und 5 kg Cellulosepulver wurden innigst miteinander vermischt, mit 17 kg Wasser unter Rühren befeuchtet und maschinell durch starkes Rühren und Kneten zu einer erdfeuchten Masse verarbeitet, die durch Hindurchpressen durch ein Sieb zu einem Granulat verarbeitet wurden. Nach Trocknen des Granulats wurde erneut gesiebt. Durch Zumischen von 1,75 kg Cellulosepulver und 0,25 kg Magnesiumstearat erhielt man ein pressfähiges Granulat.

B. Herstellung der fertigen Tabletten

Auf einer Tablettenpresse zur Herstellung von Zweischichttabletten wurden Tabletten folgender Zusammensetzung gepresst:

Untere Schicht: 190 mg Granulat Aa
(100 mg Verapamil)
Obere Schicht: 500 mg Granulat Ab
(140 mg Verapamil).

Die so erhaltenen Tabletten wurden zur Geschmacksabdeckung mit einem Filmlack aus Cellulosederivaten und üblichen Weichmachern und Farbstoffen überzogen.

*Beispiel 2*

Aa) Analog Beispiel 1 Aa wurden Granulate mit schneller Wirkstoff-Freisetzung durch Granulation von Zuckern, Cellulosen oder Stärken mit dem Wirkstoff und einem Bindemittel hergestellt. Anschliessend wurden den Granulaten Trennmittel und Fliessregulierstoffe zugesetzt. So wurden Granulate folgender Zusammensetzung erhalten:

Tabelle 1

| Substanzen | Zusammensetzung in Anteilen pro 100 | | | | | | | | |
|---|---|---|---|---|---|---|---|---|---|
| Verapamil | 45 | — | 53 | — | 53 | 50 | 60 | 40 | 80 |
| Gallopamil | — | 45 | — | 53 | — | — | — | — | — |
| Maisstärke | 20 | 20 | — | — | — | 23 | 21 | 10 | — |
| Cellulose | 34,5 | 34,5 | 20 | 20 | 23,5 | 6,5 | — | — | 5,5 |
| ®Kollidon VA 64 | — | — | 3 | 3 | 3 | — | — | 4,5 | 4 |
| ®Kollidon Cl | — | — | 3,5 | 3,5 | — | — | — | — | — |
| Magnesiumstearat | 0,5 | 0,5 | 0,5 | 0,5 | 0,5 | 0,5 | 0,5 | 0,5 | 0,5 |
| Lactose | — | — | 20 | 20 | 20 | 20 | 18,5 | 45 | 10 |

Ab) Analog Beispiel 1 Ab wurden Granulate mit verzögerter Wirkstoff-Freisetzung folgender Zusammensetzung hergestellt:

Tabelle 2

| Substanzen | Zusammensetzung in Anteilen pro 100 | | | | | | | | | |
|---|---|---|---|---|---|---|---|---|---|---|
| Verapamil | 28 | 33 | 30 | 37,5 | 37,5 | 37,5 | 37,5 | 33 | 34 | 32 |
| Natriumalginat | 50 | 43 | 58 | 50 | 50 | 50 | 50 | 44 | 48 | 62 |
| ®Kollidon 30 | 8 | 7 | 6 | 7,5 | 7,5 | 7,5 | 7,5 | 6,5 | — | — |
| Lactose | — | — | 5 | — | 4,2 | — | — | — | 14,2 | — |
| Cellulosepulver | 13,5 | 16,5 | — | — | — | 4,2 | — | — | — | — |
| Magnesiumstearat | 0,5 | 0,5 | 1 | 0,8 | 0,8 | 0,8 | 0,8 | 0,6 | 0,8 | 0,8 |
| Maisstärke | — | — | — | 4,2 | — | — | — | — | — | — |
| Polyethylenglykol | — | — | — | — | — | — | 4,2 | 3,5 | — | — |
| ®Eudragit RL | — | — | — | — | — | — | — | 12,4 | — | - |
| Methylcellulose | — | — | — | — | — | — | — | — | 3 | — |
| ®Kollidon 90 | — | — | — | — | — | — | — | — | — | 5,2 |

Tabelle 3

| Substanzen | Zusammensetzung in Anteilen pro 100 | |
|---|---|---|
| Gallopamil | 20 | 22,5 |
| Natriumalginat | 50 | 44,4 |
| ®Kollidon 30 | 7,5 | 6,6 |
| Cellulosepulver | 4,5 | 5,5 |
| Magnesiumstearat | 0,5 | 0,4 |
| Lactose | 17,5 | 10,6 |

B. Aus den in den Tabellen 1 bis 3 aufgeführten Granulaten wurden analog Beispiel 1 B Tabletten hergestellt. Dabei lagen die freien Wirkstoffmengen zwischen 60 und 150 mg und die gebundenen zwischen 80 und 190 mg bei Verapamil. Bei Gallopamil liegen die entsprechenden Werte bei 30 bis 80 bzw. 40 bis 100 mg.

*Beispiel 3*

Aus dem gemäss Beispiel 1 Ab erhaltenen Granulat wurden Presslinge mit 140 mg Verapamil hergestellt. Die so erhaltenen Presslinge wurden anschliessend in einem Presscoater mit einer Schicht Granulat gemäss Beispiel 1 Aa (Wirkstoffgehalt 120 mg Verapamil) und danach analog Beispiel 1 B mit einem Filmlack überzogen.

*Beispiel 4*

Das gemäss Beispiel 1 Ab erhaltene Granulat wurde zu Presslingen mit 150 mg Verapamil bzw. 75 mg Gallopamil gepresst. Anschliessend wurde das Granulat mit Überzügen der in Tabelle 4 angegebenen Zusammensetzung versehen, so dass die Überzüge 90 mg Verapamil bzw. 45 mg Gallopamil enthielten.

Tabelle 4

| Substanzen | Zusammensetzung in Anteilen pro 100 | | | | | |
|---|---|---|---|---|---|---|
| Verapamil | 26,3 | — | 69,5 | — | 35,4 | 45,7 |
| Gallopamil | — | 26,3 | — | 69,5 | — | — |
| Wasser | 26,3 | 26,3 | 5,2 | 5,2 | 25,8 | 20 |
| Zucker | 26,4 | 26,4 | — | — | 24,6 | 15,1 |
| Talkum | 10,5 | 10,5 | 17,4 | 17,4 | 8,9 | 12 |
| Methylcellulose | 10,5 | 10,5 | — | — | 2,2 | 3 |
| Gummi arabicum | — | — | 3,5 | 3,5 | 0,9 | 1,2 |
| ®Aerosil | — | — | — | — | 2,2 | 3 |
| Polyethylenglykol 400 | — | — | 4,4 | 4,4 | — | — |

Dieses Verfahren ist jedoch langwierig, da das Wasser entfernt werden muss. Weiterhin ist es sehr schwierig, in der Dragierschicht eine gute content uniformity zu erhalten. Die besten Ergebnisse wurden mit Überzügen erhalten, die einen geringen Wirkstoffgehalt besassen. Nach Abglätten dieser Dragees mit einer 70%igen wässrigen Zuckerlösung (pro 10 kg-Ansatz Dragees werden ca. 20 bis 40 g Zuckerlösungen benötigt) wurden die Dragees wie oben beschrieben mit einer Filmschicht überzogen.

In den Beispielen bedeuten:

| | |
|---|---|
| ®Kollidon V 64: | Mischpolymerisat aus PVP und Polyvinylacetat im Verhältnis 6 : 4 |
| ®Kollidon 30: | Kettenförmiges PVP, M 49 000 |
| ®Kollidon 90: | Kettenförmiges PVP, M 1 100 000 |
| ®Eudragit RL: | Polyacryl- und Polymetacrylsäurederivate |
| ®Aerosil: | Hochdisperses Siliciumdioxid |

**Patentansprüche**

1. Galenische Zubereitung, welche einen calciumantagonistischen Wirkstoff und gegebenenfalls weitere Wirkstoffe enthält, zur oralen Applikation mit prolongierter Wirkung zur Dauertherapie enthaltend nur die Erhaltungsdosis des Wirkstoffes an galenische Hilfsstoffe gebunden, dadurch gekennzeichnet, dass sie als calciumantagonistischen Wirkstoff Gallopamil oder Verapamil enthält und dass bei der Erhaltungsdosis das Verhältnis der Anteile mit schneller Wirkstoff-Freisetzung zu den Anteilen mit langsamer Wirkstoff-Freisetzung im Bereich von 1 : 0,6 bis 1 : 6 liegt.

2. Verfahren zur Herstellung einer galenischen Zubereitung, welche einen calciumantagonistischen Wirkstoff und gegebenenfalls weitere Wirkstoffe enthält, zur oralen Applikation mit prolongierter Wirkung zur Dauertherapie enthaltend nur die Erhaltungsdosis des calciumantagonistischen Wirkstoffes an galenische Hilfsstoffe gebunden, dadurch gekennzeichnet, dass man als calciumantagonistische Wirkstoffe Gallopamil oder Verapamil verwendet und dass man den Wirkstoff mit schneller Freisetzung und den mit langsamer Freisetzung im Verhältnis von 1 : 0,6 bis 1 : 6 in die Zubereitung einarbeitet.

**Revendications**

1. Préparation gelénique, qui contient un principe actif antagoniste du calcium et éventuellement d'autres principes actifs, ne contenant, pour l'application orale à effet prolongé en vue d'une thérapeutique de longue durée, que la dose d'entretien de principe actif liée à la matière gelénique auxiliaire, caractérisée par le fait qu'elle contient, comme principe actif antagoniste du calcium, du Gallopamile ou Verapamile et que, pour la dose d'entretien, le rapport entre la partie à libération rapide du principe actif et la partie à libération lente du principe actif est compris entre 1 : 0,6 et 1 : 6.

2. Procédé de préparation d'une préparation galénique, qui contient un principe actif antagoniste du calcium et éventuellement d'autres principes actifs, ne contenant, pour l'application orale à effet prolongé en vue d'une thérapeutique de longue durée, que la dose d'entretien de principe actif liée à la matière galénique auxiliaire, caractérisé par le fait qu'on utilise comme principe actif antagoniste du calcium, du Gallopamile ou Verapamile et qu'on fait entrer dans la préparation la partie à libération rapide du principe actif et la partie à libération lente du principe actif dans un rappaort compris entre 1 : 0,6 et 1 : 6.

**Claims**

1. A pharmaceutical composition which contains a calcium-antagonistic active compound, with or without additional active compounds, is intended for oral administration to produce a sustained action for continuous therapy and contains only the sustaining dose of active compound, bound to pharmaceutical auxiliaries, wherein the calcium-antagonistic active compound is Gallopamil or Verapamil and wherein, at the sustaining dose, the ratio of the constituents with rapid release of active compound to those with sustained release of active compound is from 1 : 0.6 to 1 : 6.

2. A process for the preparation of a pharmaceutical composition which contains a calcium-antagonistic active compound, with or without additional active compounds, is intended for oral administration to produce a sustained action for continuous therapy and contains only the sustaining dose of active compound, bound to pharmaceutical auxiliaries, wherein the calcium-antagonistic active compound is Gallopamil or Verapamil, the active compound in rapid release form and the active compound in sustained release form being incorporated into the composition in a ratio of from 1 : 0.6 to 1 : 6.